# EUROPEAN PATENT APPLICATION

(11) **EP 4 424 696 A1**
(43) Date of publication of application: **04.09.2024**
(21) Application number: 23864492.6
(22) Date of filing: 28.07.2023
(51) Int. Cl.: C07H 19/213, C07H 1/00, A61P 25/28, A61P 25/00, A61P 9/10, A61P 35/00, A61P 31/12, A61P 31/14, A61P 29/00, A61P 33/06, A61P 25/14, A61P 25/16, A61K 31/7084, A61K 31/7135

(54) **CYCLIC DINUCLEOTIDE METAL COMPOUND, AND PREPARATION METHOD THEREFOR AND USE THEREOF**

(30) Priority: 14.09.2022 CN 202211117378
(71) Applicant: Hangzhou Xingao Biotechnology Co., Ltd., Technological Development Zone, Xiaoshan District Hangzhou, Zhejiang 311200 (CN)
(72) Inventor: ZHANG, Yueru, Hangzhou, Zhejiang 311200 (CN); TAN, Yingxuan, Hangzhou, Zhejiang 311200 (CN); TAN, Xiangbao, Hangzhou, Zhejiang 311200 (CN)
(74) Representative: Vitina, Maruta
(86) International application number: PCT/CN2023/109735
(87) International publication number: WO 2024/055758

(57) **Abstract**

A cyclic dinucleotide metal compound, and a preparation and application thereof are provided. The cyclic dinucleotide metal compound is [M(cyclic dinucleotide)] or [M'₂(cyclic dinucleotide)], where M is Mg²⁺, Zn²⁺, Mn²⁺ or Fe²⁺, and M' is Li⁺. The cyclic dinucleotide is 2'3'-cGAMP, c-di-AMP, c-di-GMP, c-di-IMP, c-GMP-IMP or a derivative thereof.

## Description

### TECHNICAL FIELD

This application relates to biomedical technology, and more particularly to a cyclic dinucleotide metal compound, and a preparation and application thereof.

### BACKGROUND

In the infected mammalian cells, microorganisms and virus deoxyribonucleic acids (DNAs) can induce the endogenous and strong immune response by stimulating interferon secretion. The endoplasmic reticulum (ER) and the receptor protein (STING) are essential for immune response of cytoplasm DNAs. It has been shown that the cyclic GMP-AMP synthase (cGAS) endogenously induce the synthesis of cyclic GMP-AMP (cGAMP) under the activation condition of DNA binding. cGAMP, as the second messenger, stimulates response of the interferon INF-I by STING, and mediates the activation of the TANK-binding kinase 1 (TBK1) and the interferon regulatory factor 3 (IRF-3) to activate transcription of the type I interferon INF-β gene. The innate immunity STING pathway has been widely studied. The STING, as the target immune stimulator/agonist, has functions of anti-tumor and anti-Alzheimer's disease (anti-AD) by stimulating the type I interferon INF-β gene. However, owing to the STING agonist cGAMP is the second messenger, its short metabolism cycle *in vivo* and instability caused by easy degradation limits its pharmacodynamic effect and druggability in the clinical test. Therefore, no cGAMP-related drugs have received the market approval so far.

Alzheimer's disease (AD) has become one of the most important diseases in the world. There are nearly 50 million AD patients, but there are rare anti-Alzheimer's disease drugs approved by the US Food And Drug Administration (FDA) and China Food and Drug Administration (CFDA), and most of them can just cure the symptoms, not the disease, and their pharmacodynamic effects are not significant. Most of anti-Alzheimer's disease drugs in the world are still in the preclinical and clinical stage, and mainly act on the neuronal signaling and Amyloid-β (Aβ) plaques. However, there are many patients and a large need for drugs. The prevalence rate of AD of those aged 65 and above, doubling with each five-year increase in age, and reaching 28.9% for those aged 85 and over. The market of AD treatment in eight major countries, including the United States and Japan, reaches more than 10 billion U.S. dollars, and the market of AD drugs is increasing every year.

AD was first reported in 1907 by Alosi Alzheimer, a German scholar. The most typical pathological features of AD are the presence of the large number of amyloid plaques (senile plaques, SP), neurofibrillary tangles (NFTs), decrease of neuronal numbers and granulovacuolar degeneration. The pathogenesis of AD is complex and may be the result of the interaction of multiple factors. Up to now, an exact pathogenesis of AD is still an unsolved mystery. However, the research evidence in recent 30 years has shown that Aβ peptide, amyloid precursor protein (APP), homeostatic regulatory proteins and their related metal ion homeostasis are closely related to the occurrence and development of AD. Recent studies have reported that chronic inflammation of the brain is one of the distinctive features of AD. A groundbreaking study from the University of Bonn in Germany has found that AD is caused by inflammation of immune cells in the brain, which is undoubtedly important, because the cause and pathogenesis of AD have not been fully determined previously. Scientists predict that the discovery provides new ideas for drug development, and humans may be able to cure or even prevent AD within the next five years. The discovery has been published in Nature, a top scientific journal in the world. Prof. Michael Heneka of the University of Bonn in Germany and his colleagues believed that Aβ plaques are caused by inflammation during the process that the inflammation is involved in AD.

The cGAMP, as a natural immune agonist of STING, has been reported to show pharmacodynamic effects in AD treatment, improve learning and memory ability of AD model mice, decrease amyloid plaques in the brain and decrease chronic inflammation in the brain. However, the immune agonist cGAMP is the second messenger which is metabolized fast in vivo and seriously affecting the duration of its pharmacodynamic effect.

### SUMMARY

In view of the defects of the prior art, this application provides a cyclic dinucleotide metal compound. The cyclic dinucleotide metal compound is formed by cyclic dinucleotide and a metal ion (including lithium, magnesium, zinc, manganese, iron). The cyclic dinucleotide metal compound overcomes the defects of fast metabolism in vivo, significantly prolongs metabolism cycle in vivo and significantly increases stability in vivo. And the cyclic dinucleotide metal compound shows more significant anti-disease activity than cyclic dinucleotide in the mouse model tests, and the disease includes Alzheimer's disease, ischemic cerebrovascular injury and tumor. Therefore, the cyclic dinucleotide metal compound has a broad application prospect in the preparation of anti-disease drugs.

Technical solutions of the present disclosure are as follows.

The present disclosure provides a cyclic dinucleotide metal compound, wherein the cyclic dinucleotide metal compound is represented by [M(cyclic dinucleotide)] or [M'₂(cyclic dinucleotide)]; wherein M is Mg²⁺, Zn²⁺, Mn²⁺ or Fe²⁺; and M' is Li⁺; the cyclic dinucleotide is 2'3'-cGAMP, c-di-AMP, c-di-GMP, c-di-IMP, c-GMP-IMP or a derivative thereof.

In an embodiment, M is Mg²⁺.

The present disclosure also provides a method for preparing the cyclic dinucleotide metal compound, comprising: binding a cyclic dinucleotide anion to an ion exchange column; subjecting the ion exchange column to gradient elution with a salt solution of a target metal ion, and collecting an eluate; and subjecting the eluate to concentration, desalination, and lyophilization or cooling crystallization to obtain a target product.

The present disclosure also provides a method for treating a neurodegenerative disease in a subject in need thereof, comprising:
administering a therapeutically effective amount of the cyclic dinucleotide metal compound to the subject;
wherein the neurodegenerative disease is selected from the group consisting of Alzheimer's disease, Parkinson's disease, amyotrophic lateral sclerosis, multiple sclerosis and Huntington's disease.

In an embodiment, the cyclic dinucleotide metal compound is lithium cyclic dinucleotide.

The present disclosure also provides a method for treating an ischemic cerebrovascular injury or a craniocerebral injury in a subject in need thereof, comprising:
administering a therapeutically effective amount of the cyclic dinucleotide metal compound to the subject.

In an embodiment, the cyclic dinucleotide metal compound is magnesium cyclic dinucleotide.

The present disclosure also provides a method for treating a tumor or toxic side effects caused by an anti-tumor drug in a subject in need thereof, comprising:
administering a therapeutically effective amount of the cyclic dinucleotide metal compound to the subject.

The present disclosure also provides a method for treating a coronavirus infection disease in a subject in need thereof, comprising:
administering a therapeutically effective amount of the cyclic dinucleotide metal compound of claim 1 to the subject;
wherein the coronavirus infection disease comprises coronaviral inflammation.

The present disclosure also provides a pharmaceutical composition, comprising:
the cyclic dinucleotide metal compound; and
a pharmaceutically-acceptable excipient;
wherein a dosage form of the pharmaceutical composition is tablet, capsule, enteric-coated particle, granule, suspension, emulsion, solution, syrup or injection.

The present disclosure has the following beneficial effects.
(1) The cyclic dinucleotide metal compound of the present disclosure is a stable metal compound formed by ionic bonding between two negatively-charged phosphate radicals in the cyclic dinucleotide and the metal ion, which has improved *in-vivo* metabolic stability and increased effective concentration when used as a drug. Moreover, the cyclic dinucleotide metal compound has a simple structure, and exhibits a promising application prospect in the preparation of anti-disease drugs.
(2) The cyclic dinucleotide metal compound of the present disclosure is prepared by the ion exchange column, which is simple and easy to operate. And the obtained cyclic dinucleotide metal compound has high purity and low content of impurities, and is suitable for the preparation of related drugs.
(3) The cyclic dinucleotide metal compound of the present disclosure has significant activity in treating the neurodegenerative diseases, the ischemic cerebrovascular injury, the craniocerebral injury, the tumor and the viral infection, where the lithium cyclic dinucleotide has an outstanding effect in preventing and treating the neurodegenerative diseases, and the magnesium cyclic dinucleotide has an outstanding effect in preventing and treating the ischemic cerebrovascular injury and the craniocerebral injury.

### BRIEF DESCRIPTION OF THE DRAWINGS

Fig. 1 shows structural formulas of cyclic dinucleotide metal compounds according to an embodiment of the present disclosure.
Fig. 2 shows a nuclear magnetic resonance (NMR) spectrum of zinc cyclic dinucleotide prepared in Example 5 of the present disclosure.
Fig. 3 shows an NMR spectrum of manganese cyclic dinucleotide prepared in Example 3 of the present disclosure.
Fig. 4 shows a mass spectrum (MS) of a cyclic dinucleotide compound according to an embodiment of the present disclosure.

### DETAILED DESCRIPTION OF EMBODIMENTS

The present disclosure will be clearly and completely described below with reference to embodiments. It is obvious that the embodiments described herein are only part of embodiments of the present disclosure rather than all embodiments. And any other embodiments made by those skilled in the art based on the embodiments of the present disclosure without making creative effort shall fall within the scope of the present disclosure.

The present disclosure provides a cyclic dinucleotide metal compound represented by [M(cyclic dinucleotide)] or [M'₂(cyclic dinucleotide)], where M is a divalent metal ion, such as Mg²⁺, Zn²⁺, Mn²⁺ and Fe²⁺. In an embodiment, M is Mg²⁺ to form magnesium cyclic dinucleotide. M' is a monovalent metal ion, such as Li⁺. The cyclic dinucleotide is 2'3'-cGAMP, c-di-AMP, c-di-GMP, c-di-IMP, c-GMP-IMP or a derivative thereof. In the following embodiments, unless otherwise specified, the cyclic dinucleotide is 2'3'-cGAMP (C₂₀H₂₂N₁₀O₁₃P₂Na₂).

Regarding the application of the cyclic dinucleotide metal compound in the preparation of drugs, the drugs can be in a form of tablet, capsule, enteric-coated particle, granule, suspension, emulsion, solution, syrup or injection. And the drugs can be administered via intravenous injection, intravenous drip, hypodermic injection, intramuscular injection or direct ventricle injection.

### Preparation of 2'3'-cGAMP

The 2'3'-cGAMP (purity>98%) is synthesized under the catalysis of cyclic GMP-AMP synthase (cGAS) after activated by deoxyribonucleic acid (DNA) according to a reported method (Li P.W, et al., Immunity, 2013, 39(6), 1019-1031).

### Example 1

Provided herein was a preparation method of lithium cyclic dinucleotide, which was performed as follows.

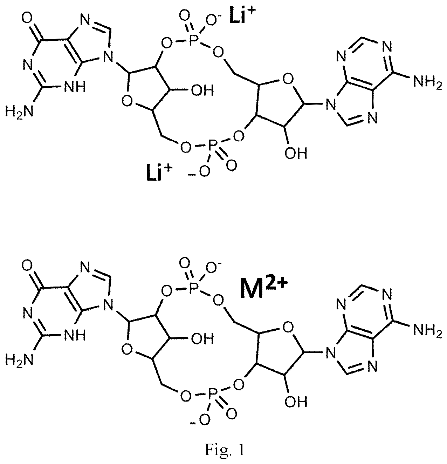

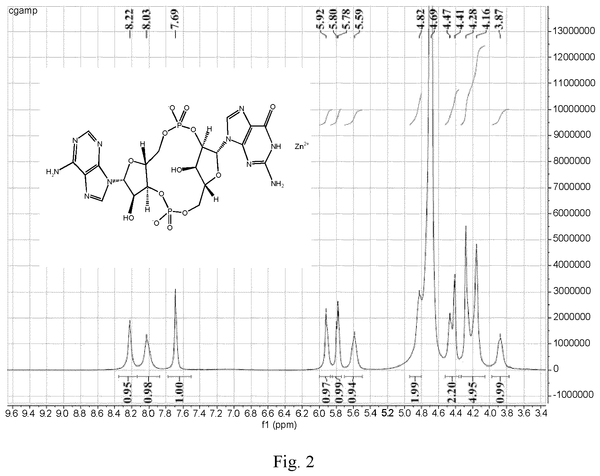

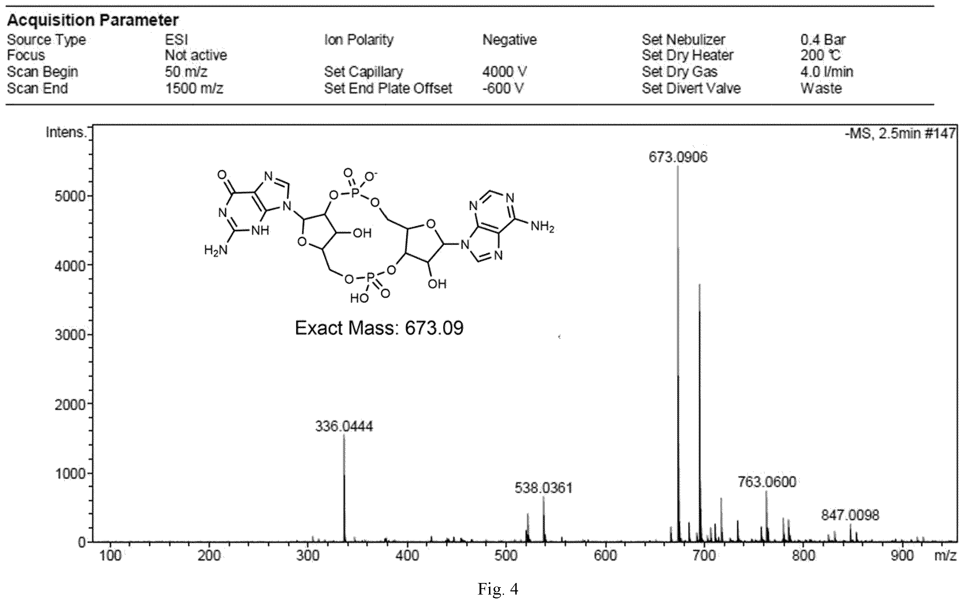

10 mmol of cyclic dinucleotide was dissolved in 1 L of pure water. By means of an AKTAPure protein purification system, the cyclic dinucleotide water solution was fed to an anion exchange column (SOURCE Q, GE Healthcare) to allow the cyclic dinucleotide to be bound to the anion exchange column. The anion exchange column was rinsed with 3 column volumes of a 20 mM lithium chloride solution, and then subjected to gradient elution with lithium chloride water solutions (with a molar concentration ranging from 50 mM to 300 mM). In this example, the analytically-pure lithium chloride was purchased from Sigma company. The lithium cyclic dinucleotide [Li₂(cGAMP)] eluate was collected, concentrated with a nanofiltration membrane concentration to filter out excess lithium chloride in the [Li₂(cGAMP)] solution. The concentrated [Li₂(cGAMP)] solution was added with ultrapure water, and concentrated again, and such a process was repeated 5 times or more to reach a volume less than 1/5 of a total volume of the original eluate. The concentrated solution was dried with a freezing dryer to give lithium cyclic dinucleotide (78% yield). A structure of the lithium cyclic dinucleotide was shown in Fig. 1. The prepared lithium cyclic dinucleotide was further subjected to metal content analysis, element analysis, nuclear magnetic resonance (NMR) spectroscopy and mass spectrometry (MS), and the analysis results were shown in Table 1, and the NMR data of lithium cyclic dinucleotide was displayed in Fig. 2, which was the same as that of the zinc cyclic dinucleotide (¹H NMR (400 MHz, D₂O) δ 8.22 (s, 1H), 8.03 (s, 1H), 7.69 (s, 1H), 5.92 (s, 1H), 5.79 (d, *J* = 7.7 Hz, 1H), 5.59 (s, 1H), 4.82 (s, 2H), 4.44 (d, *J* = 21.7 Hz, 2H), 4.22 (d, *J* = 49.6 Hz, 5H), 3.87 (s, 1H)). The electrospray ionization mass spectrometry (ESI-MS) result of the lithium cyclic dinucleotide was shown in Fig. 4, from which it could be observed that a target product peak is 673.0906 (i.e., the ion peak of cyclic dinucleotide anion), indicating that the lithium cyclic dinucleotide prepared in this example was in the form of a metal salt, and was changed into metal ions and cyclic dinucleotide ions in a solution system.

### Example 2

Main differences between this example and Example 1 were listed as follows: the target product prepared herein was magnesium cyclic dinucleotide; the eluent was a magnesium chloride solution; the collected magnesium cyclic dinucleotide eluate was concentrated, crystallized at 4 °C, and dried in a constant temperature oven to obtain the magnesium cyclic dinucleotide (81% yield). A structure of the magnesium cyclic dinucleotide was shown in Fig. 1. The magnesium cyclic dinucleotide was subjected to the metal content analysis, the element analysis, the NMR characterization and the MS characterization. The analysis result was shown in Table 1. The NMR data of the magnesium cyclic dinucleotide was basically identical to that of the lithium cyclic dinucleotide, and the MS result of the magnesium cyclic dinucleotide was consistent with that of the lithium cyclic dinucleotide.

### Example 3

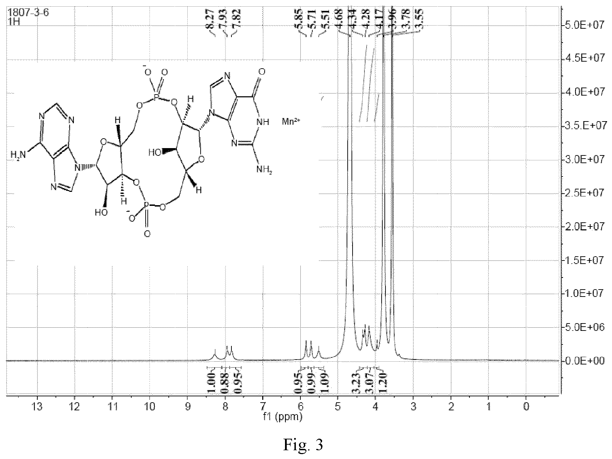

Main differences between this example and Example 1 were listed as follows: the target product prepared herein was manganese cyclic dinucleotide; the eluent was a manganese chloride solution; and the manganese cyclic dinucleotide (78% yield) was obtained. A structure of the manganese cyclic dinucleotide was shown in Fig. 1. The manganese cyclic dinucleotide was subjected to the metal content analysis, the element analysis, the NMR characterization and the MS characterization. The analysis result was shown in Table 1. Because a 3d orbital of a manganese ion has single electrons and is paramagnetic, a certain amount of ethylene diamine tetraacetic acid (EDTA) was added to remove the interference of a Mn²⁺ paramagnetic ion in the NMR characterization, and the NMR data was displayed in Fig. 3(¹H NMR (400 MHz, D₂O) δ 8.27 (s, 1H), 7.93 (s, 1H), 7.82 (s, 1H), 5.85 (s, 1H), 5.71 (s, 1H), 5.51 (s, 1H), 4.31 (d, *J* = 21.3 Hz, 3H), 4.17 (s, 3H), 3.96 (s, 1H)). And the MS result of the manganese cyclic dinucleotide was consistent with that of the lithium cyclic dinucleotide.

### Example 4

Main differences between this example and Example 1 were listed as follows: the target product prepared herein was ferrous cyclic dinucleotide; the eluent was a ferrous sulfate solution (containing a sodium hydrosulfite reductant); and the ferrous cyclic dinucleotide (75% yield) was obtained. A structure of the ferrous cyclic dinucleotide was shown in Fig. 1. The ferrous cyclic dinucleotide was subjected to the metal content analysis, the element analysis, the NMR characterization and the MS characterization. Because a 3d orbital of ferrous ion had single electrons, and is paramagnetic, a certain amount of EDTA was added to remove an interference of Fe²⁺ paramagnetic ion in the NMR characterization. The analysis result was shown in Table 1. The NMR data of the ferrous cyclic dinucleotide was basically identical to that of the manganese cyclic dinucleotide, and the MS result of the ferrous cyclic dinucleotide was consistent with that of the lithium cyclic dinucleotide.

### Example 5

Main differences between this example and Example 1 were listed as follows: the target product prepared herein was zinc cyclic dinucleotide; the eluent was a zinc chloride solution (pH was adjusted to weak acid of 6); and the zinc cyclic dinucleotide (72% yield) was obtained. A structure of the zinc cyclic dinucleotide was shown in Fig. 1. The zinc cyclic dinucleotide was subjected to the metal content analysis, the element analysis, the NMR characterization and the MS characterization. The analysis result was shown in Table 1. The NMR data of the zinc cyclic dinucleotide was basically identical to that of the lithium cyclic dinucleotide, and the MS result of the zinc cyclic dinucleotide was consistent with that of the lithium cyclic dinucleotide.

**Table 1 Analysis results of metals and elements C, H and N of cyclic dinucleotide metal compounds in Examples 1-5**

| Metal compoun d | Metal (%) | | C (%) | | H (%) | | N (%) | |
|---|---|---|---|---|---|---|---|---|
| | Experi mental value | Theore tical value | Experi mental value | Theore tical value | Experi mental value | Theore tical value | Experi mental value | Theore tical value |
| [Li₂(cGA MP)] | 1.78 | 2.04 | 34.32 | 34.93 | 2.98 | 3.20 | 20.56 | 20.38 |
| [Mg(cG AMP)] | 3.89 | 4.07 | 56.76 | 57.01 | 3.01 | 3.14 | 20.08 | 19.95 |
| [Zn(cGA MP)] | 9.05 | 8.86 | 32.18 | 32.50 | 2.67 | 2.98 | 19.12 | 18.96 |
| [Mn(cG AMP)] | 7.26 | 7.55 | 32.36 | 32.97 | 2.95 | 3.02 | 18.98 | 19.23 |
| [Fe(cGA MP)] | 7.82 | 7.68 | 37.82 | 38.41 | 2.91 | 3.01 | 19.36 | 19.20 |

### Example 6

This example provides an application for the cyclic dinucleotide metal compounds prepared in Examples 1-5 in the preparation of drugs for neurodegenerative diseases, and mainly verifies effects of the cyclic dinucleotide metal compounds on the cognitive ability of Alzheimer's disease (AD) mice.

Experimental materials, purchased from Shanghai Model Organisms Center Inc, were APP/PSI transgenic AD model mice, aged 4 months old and weighing 24-26 g. The AD model mice were randomly divided into 7 groups (10 mice in each group). The 7 groups were listed as follows: A: AD model group, as a negative control group (administration of normal saline); B: cGAMP group, as a positive control group (administration of cyclic dinucleotide); C: [Li₂(cGAMP)] group (administration of lithium cyclic dinucleotide prepared in Example 1); D: [Mg(cGAMP)] group (administration of magnesium cyclic dinucleotide prepared in Example 2); E: [Zn(cGAMP)] group (administration of zinc cyclic dinucleotide prepared in Example 5); F: [Mn(cGAMP)] group (administration of manganese cyclic dinucleotide prepared in Example 3); G: [Fe(cGAMP)] group (administration of ferrous cyclic dinucleotide prepared in Example 4). The administration in groups C-G was performed as follows. The powdered cyclic dinucleotide metal compounds were prepared into solutions of required concentration with normal saline, respectively. The administration dosage was 10 mg/kg, and the administration was performed via intraperitoneal injection once every 2 days for 60 consecutive days.

### Apparatus and method for Morris water maze test

The Morris water maze test was performed in a round pool, 1 m in diameter, 50 cm high and 30 cm deep with a white bottom of the pool. Water temperature of the Morris water maze was 23±2 °C. Four equidistant points, N, E, S and W, were marked on a pool wall as four starting points for tests. The pool was divided into four quadrants, and a platform, which was equidistant from the pool wall and a center of the pool, was placed in the third quadrant. The platform was immersed under water at a depth of 1 cm, so that the platform was not visible. A lot of reference clues (the reference clues were triangles, quadrangles, circles, and diamonds with different colors placed in each quadrant) were pasted around the pool and kept constant for the AD model mice to locate the platform.

### Place navigation test

A place navigation test was performed for 6 days, and the AD model mice were subjected to training 4 times per day at fixed time spans. At the beginning of the training, the platform was placed in the first quadrant. An AD model mouse was placed into the water facing the pool wall at any one of the four starting points. A record system was used to record time and a swimming path that the AD model mouse found the platform. The AD model mouse was placed into the water at different four starting points (in different quadrants) in the 4-time-training. The AD model mouse found the platform and rested on the platform for 10 seconds followed by next test. Or if the AD model mouse could not find the platform within 90 seconds (a latency was recorded as 90 seconds), the AD model mouse was guided to the platform by an experimenter and rested for 10 seconds followed by next test.

### Spatial probe test

24 h after the place navigation test, the platform was removed. Then the AD model mouse was placed into the water in the third quadrant, the swimming path in 180 s was recorded. Remain time and a number of the AD model mouse crossing through a location of the platform of the AD model mouse in a target quadrant (the third quadrant) were recorded to observe the spatial localization ability of the AD model mouse. SPSS10.0 software was used to process, and one-way analysis of variance (ANOVA) was adopted to compare significance of difference between the 7 groups. And test results were shown in Table 2.

**Table 2 Improvement of the cyclic dinucleotide metal compounds for cognitive ability of the AD model mice**

| Group | Percentage of time of the AD model mice crossing through the platform in the third quadrant (%) |
|---|---|
| A: AD model group | 0.38±0.21 |
| B: cGAMP group | 0.62±0.17 |
| C: [Li₂(cGAMP)] group | 0.91±0.18 |
| D: [Mg(cGAMP)] group | 0.78±0.10 |
| E: [Zn(cGAMP)] group | 0.86±0.16 |
| F: [Mn(cGAMP)] group | 0.83±0.21 |
| G: [Fe(cGAMP)] group | 0.81±0.23 |

Referring to Table 2, the group B, as the positive control group using the cyclic dinucleotide, and the groups C-G using the cyclic dinucleotide metal compounds can significantly improve the cognitive ability of the AD model mice after 60 days of drug administration. Improvement effect of the cognitive ability of the AD model mice of the groups C-G was significantly better than that of the group B. In the groups C-G, the improvement effect of the group C was significantly better than that of other groups. And an application for the lithium cyclic dinucleotide in preparing drugs for neurodegenerative diseases had significant pharmacodynamic effect, showing better improvement of the pharmacodynamic effect.

### Example 7

This example provides an application of the cyclic dinucleotide metal compounds prepared in Examples 1-5 in the preparation of drugs for prevention and treatment of neurodegenerative diseases, and mainly verifies the influence of the cyclic dinucleotide metal compounds on amyloid plaques in the brains of the AD model mice.

Differences between this example and Example 6 were as described as follow: 60 days after the drug administration in 7 groups of the AD model mice, reduction of the amyloid plaques in the brains of the AD model mice was detected. This example conducted a Thioflavin S dyeing experiment including the following steps. 60 days after the drug administration, brain tissues of the AD model mice were taken and subjected to fixing, paraffin embedding, section, dimethylbenzene dewaxing, gradient dehydration with ethanol and washing with tris buffered saline (TBS) three times. 0.3 wt.% of Thioflavin S (dissolved in 50 wt.% of ethanol) was dropped onto the brain tissues. The brain tissues were incubated for 10 min followed by washing with 50 wt.% of ethanol, PBS-washing, shade drying and sealing. A laser scanning confocal microscope (Leica, Germany) was used to detect changes of an amount of amyloid plaque deposition in the brains of the AD model mice. Experimental results were shown in Table 3.

**Table 3 Inhibitory effects of the cyclic dinucleotide metal compounds on the amyloid plaques in the brains of the AD model mice**

| Group | Average points of amyloid plaque fluorescence intensity in the brain tissues of the AD mice (field of vision) |
|---|---|
| A: AD model group | 2870±189 |
| B: cGAMP group | 1790±180 |
| C: [Li₂(cGAMP)] group | 165±75 |
| D: [Mg(cGAMP)] group | 480±80 |
| E: [Zn(cGAMP)] group | 360±75 |
| F: [Mn(cGAMP)] group | 390±87 |
| G: [Fe(cGAMP)] group | 410±91 |

Referring to Table 3, the group B, as the positive control group using the cyclic dinucleotide, and the groups C-G using the cyclic dinucleotide metal compounds can significantly reduce the amount of amyloid plaque deposition in the brains of the AD model mice. Compared to administration groups using other cyclic dinucleotide metal compounds, the improvement effect of the lithium cyclic dinucleotide group (group C) was particularly significant. And the application for the lithium cyclic dinucleotide in preparing drugs for neurodegenerative diseases showed the better improvement of the pharmacodynamic effect.

### Example 8

This example provides an application of the cyclic dinucleotide metal compounds prepared in Examples 1-5 in the preparation of drugs for treatment of ischemic cerebrovascular injury, and mainly verifies the influence of the cyclic dinucleotide metal compounds on mice with ischemic cerebrovascular injury.

Experimental animals: healthy male Institute of Cancer Research (ICR) mice, weighing 18-20 grams, purchased from Shanghai SLAC Laboratory Animal Co. Ltd, with quality certificate No. (SCXK (Shanghai) 2007-0005), and kept in clean animal houses.

Mouse model: an ischemic cerebrovascular injury model was prepared by a middle cerebral artery occlusion (MCAO) method to verify therapeutic effect of the cyclic dinucleotide metal compounds on ischemic cerebrovascular diseases in the experimental animals.

Experimental method: the ICR mice were anesthetized with 10 wt.% of chloral hydrate by intraperitoneal injection, a mid-cervical incision was made to isolate and ligate a proximal segment, a carotid artery and a branch vessel of a right common carotid artery. A right internal carotid artery was isolated, a pterygoid artery was isolated down along the right internal carotid artery followed by root ligation. A wire was prepared on the proximal segment, and an arterial clip was placed on a distal segment. An incision was made at a branch of the right common carotid artery, and a nylon wire was tied in. A monofilament entered the internal carotid artery, a cranium to an anterior cerebral artery to block all sources of blood flow in a middle cerebral artery. The arterial clip was removed, and the wire prepared on the proximal segment was tied in followed by skin suture. The experimental animals were subjected to cage culture after operation. 2 h after the ischemia, the experimental animals were subjected to abdominal administration by injection, and the nylon wire was removed for reperfusion. 8 h after the reperfusion, the experimental animals were subjected to re-administration. After the operation, behavioral score was performed using a single-blind method with reference to the Zea Longa 5-point scoring system, which was shown as follows: 0 point, normal performance with no neurological deficits; 1 point, contralateral forepaws could not fully extend; 2 points, circling to the opposite side when walking; 3 points, falling to the opposite side; 4 points, no spontaneous walking and loss of consciousness.

In the experiment, the ICR mice were divided into 7 groups (10 mice in each group). The 7 groups were listed as follows: A: AD model group, as a negative control group (administration: normal saline); B: cGAMP group, as a positive control group (administration: cyclic dinucleotide); C: [Li₂(cGAMP)] group (administration: lithium cyclic dinucleotide prepared in Example 1); D: [Mg(cGAMP)] group (administration: magnesium cyclic dinucleotide prepared in Example 2); E: [Zn(cGAMP)] group (administration: zinc cyclic dinucleotide prepared in Example 5); F: [Mn(cGAMP)] group (administration: manganese cyclic dinucleotide prepared in Example 3); G: [Fe(cGAMP)] group (administration: ferrous cyclic dinucleotide prepared in Example 4); where a preparation method for administration in groups C-G were as follows. Powders prepared in the groups C-G were prepared to solutions of required concentration with normal saline. The administration dosage of every group was 10 mg/kg. And the ICR mice were administered by intraperitoneal injection. The ICR mice were administered once after 2 h of ischemia, then the nylon wire was removed for reperfusion. 8 h after the reperfusion, the ICR mice were administered once. After 24 h, the ICR mice were administered once a day for 7 consecutive days. Experimental results of the 7 groups were shown in Table 4.

**Table 4 Behavioral score result of ischemic cerebrovascular injury treatment by the cyclic dinucleotide metal compounds**

| Group | Score (24 h after operation) | Score (7h after operation) |
|---|---|---|
| A: AD model group | 3.50±0.28 | 2.96±0.24 |
| B: cGAMP group | 2.15±0.32 | 1.67±0.41 |
| C: [Li₂(cGAMP)] group | 1.82±0.19 | 1.49±0.26 |
| D: [Mg(cGAMP)] group | 1.18±0.24 | 1.09±0.18 |
| E: [Zn(cGAMP)] group | 1.57±0.38 | 1.31±0.15 |
| F: [Mn(cGAMP)] group | 1.62±0.39 | 1.38±0.27 |
| G: [Fe(cGAMP)] group | 1.67±0.35 | 1.41±0.29 |

Referring to Table 4, the groups B-G with administration by intraperitoneal injection can improve the behavioral score of the mice with ischemic cerebrovascular injury. Compared to the group B, the groups C-G had more significant pharmacodynamic effect on ischemic cerebrovascular injury. Among them, improvement effect of the magnesium cyclic dinucleotide (group D) was significant. And an application for the magnesium cyclic dinucleotide in preparing drugs for ischemic cerebrovascular injury had significant improvement of pharmacodynamic effect.

### Example 9

This example provides an application for the cyclic dinucleotide metal compounds prepared in Examples 1-5 in preparing the drugs for ischemia-reperfusion injury (IRI), and mainly verifies the influence of the cyclic dinucleotide metal compounds on mice IRI.

Experimental animals: healthy male mice, weighing 280-300 grams, purchased from Shanghai SLAC Laboratory Animal Co. Ltd, with quality certificate No. (SCXK (Shanghai) 2007-0005), and kept in clean animal houses.

Establishment of a mouse cerebral ischemia model of mice and experimental method: 280-300 grams of male standard deviation (SD) mice were selected for experiments. The male SD mice were subjected to fasting without water deprivation for 12h before operation followed by weighing, numbering and random grouping. The male SD mice were anesthetized by intraperitoneal injection of 10 wt.% chloral hydrate at a dosage of 0.3 mL/100 g. After the male SD mice had no pain reflexes, they were subjected to neck skin preparation followed by being fixed on a dissecting table in supine position. After neck skins were disinfected with sterilized cotton balls, about 1 cm of the neck skins were cut in mid-cervical to expose subcutaneous tissues, and subcutaneous fat and the fascia were bluntly plucked with curved tweezers to expose neck muscle groups. The common carotid arteries and their upwardly internal and external carotid branches were visualized by opening the digastric muscle. The external, internal, and common carotid arteries were carefully dissected, and a thin wire was threaded under each of the three arteries, and homemade plastic spacers were placed under the common carotid arteries. The external carotid arteries and proximal ends of the common carotid arteries were ligated. After the internal common carotid arteries were clamped by arterial clips, the common carotid arteries were stuck into with homemade curved hooks followed by picking up and leading in monofilaments. The spacers were removed, and the monofilaments were gently pushed into the internal common carotid arteries and craniums. When black marks of the monofilaments (about 18 mm) exceeded intersections of the internal carotid arteries and external carotid arteries, obvious resistance can be felt, indicating that heads of the monofilaments had passed intersections of middle cerebral arteries and anterior cerebral arteries and entered the anterior cerebral arteries. At this time, blood supplies of the middle cerebral arteries from the anterior cerebral arteries were blocked. Thin wires under the internal carotid arteries were ligated to secure the monofilaments to prevent the monofilaments from retreating. Excess thin wires were cut, penicillin was applied to wounds to prevent infection. The male SD mice were sutured and placed under a warm lamp to keep warm. Timing was performed when blood flows of the middle cerebral arteries were blocked by the monofilaments. After 2 hours, the monofilaments were gently pulled out and the black marks were seen, then reperfusion can be achieved.

Triphenyl tetrazolium chloride (TTC) dyeing: After being injured by 2 h cerebral ischemia and 24 h reperfusion, the male SD mice were anesthetized with 0.3 mL/100 g dosage of 10 wt.% of chloral hydrate by intraperitoneal injection and subjected to abdominal aorta bleeding to reduce influence of blood in brains. Then whole heads of the male SD mice were removed immediately after decapitating, and washed with normal saline followed by being promptly put into a -20 °C refrigerator to freeze for 20min. Frozen brains were collected and subjected to coronal sections, each 2 mm of thickness, by a blade. Then the coronal sections were subjected to dark dyeing in a 2 wt.% of TTC solution for 20 min and fixing in a 4 wt.% of polyformaldehyde solution for 6 h followed by photographing. Then, Photoshop software was used to define area and calculate, and a formula was represented as a percentage of cerebral ischemic area = a total area of white area / a total area of ischemic hemisphere × 100%.

Drug preparation and experimental grouping: Edaravone was used as a positive control drug, and an Edaravone injection was 30 mg/20 mL. A single dosage for each of the male SD mice was 0.4 mL/100 g, that is, 6 mg/kg. The cyclic dinucleotide metal compounds prepared in Examples 1-5 were prepared into 0.75 mg/mL injection drugs with the normal saline. Each of the male SD mice was subjected to caudal vein injection with 0.4 mL/100 g dosage of drugs, that is, 5 mg/kg. The male SD mice were divided into 8 groups (10 mice in each group). The 8 groups were listed as follows: model group: normal saline, 4 mg/kg of the caudal vein injection; positive control group: Edaravone, 6 mg/kg of the caudal vein injection; Group I: cyclic dinucleotide, 5 mg/kg of the caudal vein injection; Group II: [Li₂(cGAMP)], 5 mg/kg of the caudal vein injection; Group III: [Mg(cGAMP)], 5 mg/kg of the caudal vein injection; Group IV: [Zn(cGAMP)], 5 mg/kg of the caudal vein injection; Group V: [Mn(cGAMP)], 5 mg/kg of the caudal vein injection; and Group VI: [Fe(cGAMP)], 5 mg/kg of the caudal vein injection. Each of the male SD mice in administration groups was subjected to caudal vein injection after 30 min ischemia. TTC dyeing results of brain tissues of the male SD mice in each group were counted. And the TTC dyeing results were shown in Table 5.

**Table 5 Therapeutic effects of the cyclic dinucleotide metal compounds on the IRI mice**

| Group | Average value of percentage of cerebral ischemic area (%) |
|---|---|
| Model group | 46.89 |
| Positive control group | 28.97 |
| Group II cGAMP | 26.86 |
| Group II [Li₂(cGAMP)] | 25.25 |
| Group III [Mg(cGAMP)] | 18.16 |
| Group IV [Zn(cGAMP)] | 23.52 |
| Group V [Mn(cGAMP)] | 25.56 |
| Group VI [Fe(cGAMP)] | 26.78 |

Referring to Table 5, the percentage of cerebral ischemic area of the positive control group and groups I-VI was significantly reduced than that of the model group, showing that the cyclic dinucleotide metal compounds had protective effect on the IRI mice. At the same time, in group I-VI, the magnesium cyclic dinucleotide (group III) had the best protective effect on the IRI mice, which was significantly better than the positive control group and other cyclic dinucleotide metal compounds.

### Example 10

This example provides an application of the cyclic dinucleotide metal compounds prepared in Examples 1-5 in the preparation of anti-tumor drugs, and mainly verifies inhibitory effect of the cyclic dinucleotide metal compounds on growth of transplanted tumor in mice.

Experimental animals: BALB/c ordinary mice and C57BL/6 ordinary mice, male, weighing 20-22 grams, 7-8 weeks old, purchased from Shanghai SLAC Laboratory Animal Co. Ltd.

Feeding condition: All mice were free to feed and drink at room temperature (23±2) °C). Feeds and water were subjected to high-pressure sterilization in advance, and all experimental feeding processes were at specified pathogen free (SPF) level.

Tumour cell line: mouse colorectal cancer cell line CT26 and mouse breast cancer cell line 4T1, both purchased from Chinese Academy of Sciences Cell Bank.

Establishment and intervention of tumor model mice: cells were subjected to culture and subculture, collected in a logarithmic phase and prepared as 1.0× 10⁷/mL of cell suspension. 0.2 mL of the cell suspension was injected armpits of forelegs of the mice (with a cell number of 2.0×10⁶), and tumor transplantation was successful in about 7 days. Mice with successful tumor transplantation were randomly divided into 7 groups (10 mice in each group). The 7 groups were listed as follows: A: negative control group (injection with normal saline); B: positive control cGAMP group (10 mg/kg of injection); C: [Li₂(cGAMP)] group (10 mg/kg of injection); D: [Mg(cGAMP)] group (10 mg/kg of injection); E: [Zn(cGAMP)] group (10 mg/kg of injection); F: [Mn(cGAMP)] group (10 mg/kg of injection); and G: [Fe(cGAMP)] group (10 mg/kg of injection). The mice with successful tumor transplantation were subjected to administration by intraperitoneal injection with a volume of 200 µL per mice once every 2 days for 20 days. After 20 days, the mice with successful tumor transplantation were killed, and the tumor were weighed. A formula of inhibitory rate of tumor was as represented as inhibitory rate of tumor = [1-average tumor weight of experimental group/average tumor weight of the negative control group] × 100%.

According to the establishment of tumor model mice, the mouse colorectal cancer cell line CT26 was cultured and transplanted to the BALB/c ordinary mice, and the mouse breast cancer cell line 4T1 was cultured and transplanted to the BALB/c ordinary mice. Anti-tumor effects on different drugs were observed.

Statistical analysis: data was expressed as mean ± standard deviation and processed by the SPSS10.0 software. One-way ANOVA was used to detect and compare significance of tumor weights in each group. Statistical analysis results were shown in Table 6 and Table 7.

**Table 6 Inhibitory effects of the cyclic dinucleotide metal compounds on the mouse colorectal cancer cell line CT26 of BALB/c ordinary mice by subcutaneous transplantation**

| Group | Average tumor weight (g) | Average inhibitory rate of the tumor (%) |
|---|---|---|
| Negative control group | 1.825±0.146 | / |
| Positive cGAMP group | 0.712±0.145** | 61.0 |
| C: [Li₂(cGAMP)] group | 0.675±0.163** | 63.0 |
| D: [Mg(cGAMP)] group | 0.635±0.178** | 65.2 |
| E: [Zn(cGAMP)] group | 0.201±0.123** | 88.9 |
| F: [Mn(cGAMP)] group | 0.258±0.105** | 85.8 |
| G: [Fe(cGAMP)] group | 0.301±0.112** | 83.5 |

| | | |
|---|---|---|
| Note: *P<0.05 vs the negative control group; and **P<0.01 vs the negative control group. | | |

**Table 7 Inhibitory effect of the cyclic dinucleotide metal compounds on the mouse breast cancer cell line 4T1 of BALB/c ordinary mice by subcutaneous transplantation**

| Group | Average tumor weight (g) | Average inhibitory rate of tumor (%) |
|---|---|---|
| Negative control group | 1.846±0.165 | / |
| Positive cGAMP group | 0.761±0.157** | 58.8 |
| C: [Li₂(cGAMP)] group | 0.712±0.146** | 61.5 |
| D: [Mg(cGAMP)] group | 0.679±0.183** | 63.2 |
| E: [Zn(cGAMP)] group | 0.212±0.135** | 89.1 |
| F: [Mn(cGAMP)] group | 0.287±0.116** | 84.9 |
| G: [Fe(cGAMP)] group | 0.301±0.116** | 83.6 |

| | | |
|---|---|---|
| Note: *P<0.05 vs the negative control group; and **P<0.01 vs the negative control group. | | |

Referring to Table 6 and Table 7, in the transplantation tumor model prepared by mouse subcutaneous transplantation, the cyclic dinucleotide metal compounds can significantly inhibit the growth of transplanted tumor. 21 days after the administration, tumor weights of the cyclic dinucleotide metal compounds were significantly lower than that of the negative control group (P<0.05, P<0.01), indicating that groups of the cyclic dinucleotide metal compounds are better than the positive cGAMP group. And the [Zn(cGAMP)] group and the [Mn(cGAMP)] group had significantly improved anti-tumor effects.

### Example 11

This example provides an application for the cyclic dinucleotide metal compounds prepared in Examples 3 and 5 in preparing drugs for preventing toxic side effects caused by anti-tumor drugs, and mainly verifies reduction effect of the cyclic dinucleotide metal compounds on toxic side effects of the anti-tumor drugs.

Experimental animals: BALB/c ordinary mice, male, weighing 20-22 grams, 7-8 weeks old, at SPF level, purchased from Shanghai SLAC Laboratory Animal Co. Ltd.

Feeding condition: All mice were free to feed and drink at room temperature (23±2) °C). Feeds and water were subjected to pressure sterilization, and all experimental feeding processes were at the SPF level.

Tumour cell line herein was the mouse colorectal cancer cell line CT26 purchased from Chinese Academy of Sciences Cell Bank.

Establishment and intervention of tumor model mice: cells were subjected to culture and subculture, collected in the logarithmic phase and prepared as 1.0×10⁷/mL of cell suspension. 0.2 mL of the cell suspension was injected armpits of forelegs of the mice (with a cell number of 2.0×10⁶), and tumor transplantation was successful in about 7 days. The mice were randomly divided into 5 groups (10 mice in each group). The 5 groups were as follows: A: negative control group (injection with normal saline); B: positive control oxaliplatin group (5 mg/kg of injection); C: oxaliplatin (5 mg/kg of injection)+cGAMP (10 mg/kg of injection); D: oxaliplatin (5 mg/kg of injection)+[Mn(cGAMP)] group (10 mg/kg of injection); and E: oxaliplatin (5 mg/kg of inj ection)+[Zn(cGAMP)] group (10 mg/kg of injection). The mice were subj ected to administration by intraperitoneal injection with the volume of 200 µL per mice once every 2 days for 20 days. After 20 days, the mice were weighed and their blood was collected. Then the mice were killed, and the tumor were weighed. The formula of inhibitory rate of tumor was represented as inhibitory rate of tumor = [1-average tumor weight of experimental group/average tumor weight of the negative control group] × 100%.

### Result analysis

(1) Influence of the cyclic dinucleotide metal compounds on weight and a survival rate of the mice: a percentage of weight loss, the survival rate and an average inhibitory rate of tumor of the mice after 20 days administration were counted, and the statistical result was shown is Table 8.

**Table 8 Percentage of weight loss, survival rate and average inhibitory rate of tumor of the mice**

| Group | Weight loss (%) | Survival rate (%) | Inhibitory rate (%) |
|---|---|---|---|
| A: Negative control group | / | 100 | / |
| B: Positive control oxaliplatin group | 40 | 50 | 78 |
| C: oxaliplatin+cGAMP | 10 | 90 | 84 |
| D: oxaliplatin+[Mn(cGAMP)] | 8 | 100 | 92 |
| E: oxaliplatin+[Zn(cGAMP)] | 6 | 100 | 95 |

Referring to Table 8, the cyclic dinucleotide metal compounds [Mn(cGAMP)] and [Zn(cGAMP)] significantly reduced toxic side effects of platinum complexes. Weights of the positive control oxaliplatin group lost by 40%. Combinations of oxaliplatin and the cyclic dinucleotide metal compounds had much less toxic side effects than that of oxaliplatin, and significantly improved the survival rate and average inhibitory rate. And the manganese cyclic dinucleotide and the zinc cyclic dinucleotide had significant improvement effects on toxic side effects of the anti-tumor drugs.
(2) Reduction of hematologic toxicity of the anti-tumor drugs by the cyclic dinucleotide metal compounds: peripheral blood of the mice in each group was collected, and the results of blood tests were compared. And the results were shown in Table 9.

**Table 9 Results of blood tests of the peripheral blood of the mice in each group**

| Group | Red blood cells ×10⁶/µL | Hemoglobin g/L | Platelets ×10³/µL | White blood cells×10³/µL |
|---|---|---|---|---|
| A: Negative control group | 7.92±1.23 | 130±2 | 581±36 | 2.78±0.36 |
| B: Positive control oxaliplatin group | 4.51±0.65 | 88±9 | 368±56 | 3.87±0.08 |
| C: Oxaliplatin + cGAMP | 6.37±0.18 | 132±6 | 758±97 | 2.86±0.37 |
| D: Oxaliplatin + [Mn(cGAMP)] | 7.83±0.21 | 136±5 | 726±92 | 2.69±0.32 |
| E: Oxaliplatin + [Zn(cGAMP)] | 8.26±0.31 | 134±7 | 723±53 | 3.16±0.58 |

Referring to Table 9, the content of the red blood cells and the number of the hemoglobin of the positive control oxaliplatin group were significantly lower than a normal value of the negative control group. Blood test indexes of a combination of the cGAMP and the oxaliplatin were significantly better than that of the positive control oxaliplatin group, which were in a normal range and is consistent with normal data of the negative control group, indicating that [Mn(cGAMP)] and [Zn(cGAMP)] can significantly reduce the hematologic toxicity of the anti-tumor drugs.
(3) Reduction of toxicity of the anti-tumor drugs on livers and kidneys of the mice by the cyclic dinucleotide metal compounds: a content of alanine transaminase (ALT) and Creatinine in serum of the mice in each group was counted. Counted results were shown in Table 10.

**Table 10 Content statistics of the ALT and Creatinine in serum of the mice in each group**

| Group | ALT (U/L) | Creatinine (µmol/L) |
|---|---|---|
| Ordinary mouse group (reference value) | 81.45±9.16 | 36.56±3.02 |
| A: Negative control group | 86.78±3.86 | 38.26±0.25 |
| B: Positive control oxaliplatin group | 118.25±7.84 | 80.33±4.65 |
| C: Oxaliplatin + cGAMP | 88.05±0.56 | 39.28±4.36 |
| D: Oxaliplatin + [Mn(cGAMP)] | 81.25±6.85 | 34.46±3.89 |
| E: Oxaliplatin + [Zn(cGAMP)] | 82.46±7.35 | 35.46±4.16 |

Referring to Table 10, compared to the ordinary mouse group, the content of ALT and Creatinine of the positive control oxaliplatin group exceeded the normal value in liver function index. Indexes of a combination of the cyclic dinucleotide metal compounds and oxaliplatin were consistent with that of the ordinary mouse group, and showed no significant change. Oxaliplatin damaged the livers and kidneys of the mice, and the manganese cyclic dinucleotide and the zinc cyclic dinucleotide can significantly reduce the toxicity of the anti-tumor drugs on the livers and kidneys.

### Example 12

This example provides an application for the cyclic dinucleotide metal compounds prepared in Examples 1-5 as immunologic adjuvants in preparing antiviral vaccines, and mainly verifies immunologic adjuvant functions of the cyclic dinucleotide metal compounds.

Experimental animals: BALB/c ordinary mice, male, weighing 20-22 grams, 7-8 weeks old, at the SPF level, and purchased from Shanghai SLAC Laboratory Animal Co. Ltd.

Feeding condition: All mice were free to feed and drink at room temperature (23±2) °C). Feeds and water were subjected to high-pressure sterilization, and all experimental feeding processes were at the SPF level.

Mouse immunization: The mice were randomly divided into 8 groups (10 mice in each group). The 8 groups were listed as follows: A: negative control; B: ovalbumin (OVA) + aluminum adjuvant; C: OVA + cyclic dinucleotide; D: OVA+ [Li₂(cGAMP)]; E: OVA + [Mg(cGAMP)]; F: OVA + [Zn(cGAMP)]; G: OVA + [Mn(cGAMP)]; and H: OVA + [Fe(cGAMP)]. Each mouse in the groups B-H was subjected to subcutaneous injection with 10 µg of OVA and 100 µg of the aluminum adjuvant or the cyclic dinucleotide metal compounds, and each mouse in the group A was subjected to injection with the normal saline. The mice were respectively immunized once on days 1, 7, and 14, and bronchoalveolar lavage fluids and blood samples were collected on day 2. Enzyme linked immunosorbent assay was used to measure valences of antibodies induced by the cyclic dinucleotide metal compounds as the immunologic adjuvants. Experimental results were shown in Table 11.

**Table 11 Immunologic adjuvant valences of the cyclic dinucleotide metal compounds**

| Group | Serum IgG (Iog2 value) | Bronchoalveolar lavage fluid IgA (Iog2 value) |
|---|---|---|
| A: Negative control group | 10.5±1.3 | 1.2±0.3 |
| B: OVA+aluminum adjuvant | 21.5±1.7 | 2.6±0.4 |
| C: OVA+cGAMP | 20.7±0.9 | 2.7±0.2 |
| D: OVA+[Li2(cGAMP)] | 19.8±1.3 | 2.5±0.6 |
| E: OVA+[Mg(cGAMP)] | 22.3±1.7 | 3.3±0.5 |
| F: OVA+[Zn(cGAMP)] | 31.6±1.3 | 5.4±0.7 |
| G: OVA+[Mn(cGAMP)] | 29.8±1.9 | 5.9±0.8 |
| H: OVA+[Fe(cGAMP)] | 24.6±1.6 | 5.1±0.7 |

Referring to Table 11, as the immunologic adjuvants, the cyclic dinucleotide metal compounds can significantly induce immune antibodies, and effects of the cyclic dinucleotide metal compounds were significantly better than that of the aluminum adjuvant. And [Mg(cGAMP)] and [Zn(cGAMP)] had significant effects of the immunologic adjuvant valences, and had significantly improved than the cyclic dinucleotide and other cyclic dinucleotide metal compounds.

### Example 13

This example provides an application for the cyclic dinucleotide metal compounds prepared in Examples 1-5 in preparing drugs for viral inflammation, and mainly verifies functions of acquired immunity induced by the cyclic dinucleotide metal compounds.

Experimental animals: C57BL/6 ordinary mice, male, weighing 20-22 grams, 7-8 weeks old, purchased from Shanghai SLAC Laboratory Animal Co. Ltd.

Feeding condition: All mice were free to feed and drink at room temperature (23±2) °C). Feeds and water were subjected to pressure sterilization, and all experimental feeding processes were at the SPF level.

The mice were randomly divided into 8 groups (10 mice in each group). The 8 groups were as listed follows: A: Negative control group (isotype control antibody group); B: blank group; C: cyclic dinucleotide group; D: [Li₂(cGAMP)] group; E: [Mg(cGAMP)] group; F: [Zn(cGAMP)] group; G: [Mn(cGAMP)] group; and H: [Fe(cGAMP)] group. Isotype control flow antibody was purchased from eBiosciences, antibody magnetic strain was purchased from Militeny Biotech, and flow cytometry was purchased from BD company. After 14 days of immunization (immunization process was the same as that in the Example 12), spleens and lung tissues of the mice were collected, respectively mashed, filtered through 40 micron pores followed by 1000rpm of centrifugation for 10 min to solate unlysed immune cells. Dendritic cells (DCs) (CD40\CD80\CD86\MHCII), T(CD8+) cells were separated, and added with corresponding FAC antibodies (diluted with Fluorescence-Activated Cell Sorting (FACS) buffer). Isotype control antibody was used as negative control. After antibody addition, the DCs and T (CD8+) cells were incubate for 1 h, centrifuged and washed by phosphate buffer saline (PBS) followed by analysis by the flow cytometry to sort suitable cells. Fluorescence intensity (MFI) of sorted cells was measured. Flow cytometry results were shown in Table 12.

**Table 12 Activation effects of the cyclic dinucleotide metal compounds on inducing immune cells**

| Group | Spleen (CD40/CD80/CD86/MGCII) | Spleen (% CD8+T cells) | Lung (% CD8+T cells) |
|---|---|---|---|
| A: Isotype control antibody group | 5.2/3.2/1.3/7.8 | 0.7±0.3 | 0.2±0.1 |
| B: Blank group | 36.8/15.5/5.3/122.6 | 0.8±0.4 | 0.3±0.2 |
| C: cGAMP | 120.5/112.1/16.5/208.1 | 2.2±0.2 | 0.5±0.2 |
| D: [Li₂(cGAMP)] | 136.2/157.6/21.7/250.8 | 3.1±0.3 | 0.6±0.2 |
| E: [Mg(cGAMP)] | 156.6/234.6/36.8/338.2 | 6.2±0.6 | 1.7±0.3 |
| F: [Zn(cGAMP)] | 264.7/245.7/42.6/424.5 | 8.6±1.8 | 1.6±0.2 |
| G: [Mn(cGAMP)] | 255.9/132.6/26.3/276.3 | 7.5±1.3 | 0.8±0.3 |
| H: [Fe(cGAMP)] | 236.8/134.6/24.1/247.1 | 6.9±1.2 | 0.7±0.3 |

Referring to Table 12, the cyclic dinucleotide metal compounds can significantly activate the DCs and T cells. And [Mg(cGAMP)] and [Zn(cGAMP)] had significant effects of pharmacodynamic effects of the acquired immunity, and had significantly improved than the cyclic dinucleotide and the cyclic dinucleotide metal compounds.

### Research of acute toxicity of the cyclic dinucleotide metal compounds

Experimental materials: 20 ICR mice (purchased from Shanghai SLAC Laboratory Animal Co. Ltd), half male and half female, weighing 20-22 g. The ICR mice were fed with pellet feeds and free to eat and drink water.

Experimental method: The ICR mice were subjected to intraperitoneal injection with 1 g/kg of cyclic dinucleotide metal compound drugs (prepared with normal saline for injection). Toxic reaction and death of the ICR mice in 14 days after administration were observed. The results showed that activity of the ICR mice was normal after the intraperitoneal injection. No death occurred in the ICR mice within 14 days after administration. On the 15th day, all ICR mice were killed and dissected, and no obvious lesions were found in all organs by visual examination.

Experimental result: The results above showed that maximum tolerated dose (MTD) of the intraperitoneal injection was not less than 1 g/kg, indicating that the acute toxicities of the cyclic dinucleotide metal compound drugs was low.

### Stability testes of the cyclic dinucleotide metal compounds in blood

Experimental animals: SD mice, weighing 200-220 g, were purchased from Shanghai SLAC Laboratory Animal Co. Ltd.

Feeding condition: All mice were free to feed and drink at room temperature 25±2 °C. Feeds and water were subjected to pressure sterilization, and all experimental feeding processes were at the SPF level.

Main test steps: The SD mice were randomly divided into 7 groups (10 mice in each group). The 7 groups were listed as follows: blank control group (injection of normal saline); cDAMP group; [Li₂(cGAMP)] group; [Mg(cGAMP)] group; [Zn(cGAMP)] group; [Mn(cGAMP)] group; and [Fe(cGAMP)] group. At fixed time points (0 h, 1 h, 2 h, 4 h, 6 h, 8 h, 10 h, 12 h, 14 h, 16 h, 20 h, and 24 h), 1 mL of blood of each of the SD mice was taken through ophthalmic vein by a capillary, placed in a 1.5 mL of centrifuge tube followed by centrifugation for 30 min. Upper serum was collected and stored at -80°C for use. Concentrations of cGAMP and IFN-β in the serum were determined by ELISA kit. Based on IFN-β induced by activation of innate immunity paths of the cGAMP and the cyclic dinucleotide metal compounds, a content of the IFN-β in the blood was detected, and stability and activation activity of immune path of the cGAMP and the cyclic dinucleotide metal compounds were evaluated. Concentration values (including cGAMP and IFN-(3) in blood samples of the SD mice in each group were detected by the ELISA kit, and the concentration values and average values at each time point were calculated. A concentration change curve over time was drawn, and a peak of the concentration change curve was obtained. The results were shown in Table 13.

**Table 13 Stability of the cyclic dinucleotide metal compounds in blood and a content of the IFN-β induced by the cyclic dinucleotide metal compounds**

| Group | Average peak concentration of cGAMP blood sample (h) | Average peak concentration of IFN-β blood sample (h) |
|---|---|---|
| Blank control group | / | / |
| cGAMP control group | 2.0 | 4.0 |
| [Li₂(cGAMP)] group | 4.1 | 5.6 |
| [Mg(cGAMP)] group | 4.3 | 5.9 |
| [Mn(cGAMP)] group | 4.7 | 6.0 |
| [Zn(cGAMP)] group | 4.8 | 6.2 |
| [Fe(cGAMP)] group | 4.5 | 5.6 |

Referring to Table 13, cGAMP concentration in blood of the SD mice and time of average peak concentration of IFN-β biological effect induced by the cGAMP had significant changes. The cyclic dinucleotide metal compounds had significant improvement effect on metabolic cycle than that of the cGAMP, indicating that compared with the cyclic dinucleotide, the cyclic dinucleotide metal compounds were more stable, had longer metabolic cycles in blood, and had more significantly improve pharmaceutic effect. Among the tested cyclic dinucleotide metal compounds, [Mg(cGAMP)] and [Zn(cGAMP)] exhibited better drug stability and higher effective concentration.

Described above are only preferred embodiments of the present disclosure, which are not intended to limit the present disclosure. It should be note that various modifications, variations and replacements made by those skilled in the art without departing from the scope and spirit of the present disclosure shall fall within the scope of this application defined by the appended claims.

## Claims

1. A cyclic dinucleotide metal compound, **characterized in that** the cyclic dinucleotide metal compound is represented by [M(cyclic dinucleotide)] or [M'₂(cyclic dinucleotide)];
wherein M is Mg²⁺, Zn²⁺, Mn²⁺ or Fe²⁺; M' is Li⁺; and the cyclic dinucleotide is 2'3'-cGAMP, c-di-AMP, c-di-GMP, c-di-IMP, c-GMP-IMP or a derivative thereof.

2. The cyclic dinucleotide metal compound according to claim 1, wherein M is Mg²⁺.

3. A method for preparing the cyclic dinucleotide metal compound according to claim 1 or 2, comprising:
binding a cyclic dinucleotide anion to an ion exchange column;
subjecting the ion exchange column to gradient elution with a salt solution of a target metal ion, and collecting an eluate; and
subjecting the eluate to concentration, desalination, and lyophilization or cooling crystallization to obtain the cyclic dinucleotide metal compound.

4. The cyclic dinucleotide metal compound of claim 1 for use in the treatment of a neurodegenerative disease, **characterized in that** the neurodegenerative disease is selected from the group consisting of Alzheimer's disease, Parkinson's disease, amyotrophic lateral sclerosis, multiple sclerosis and Huntington's disease.

5. The cyclic dinucleotide metal compound for use according to claim 4, **characterized in that** the cyclic dinucleotide metal compound is lithium cyclic dinucleotide.

6. The cyclic dinucleotide metal compound of claim 1 for use in the treatment of an ischemic cerebrovascular injury or a craniocerebral injury.

7. The cyclic dinucleotide metal compound for use according to claim 6, **characterized in that** the cyclic dinucleotide metal compound is magnesium cyclic dinucleotide.

8. The cyclic dinucleotide metal compound of claim 1 for use in the treatment of a tumor or toxic side effects caused by an anti-tumor drug.

9. The cyclic dinucleotide metal compound of claim 1 for use in the treatment of a coronavirus infection disease, **characterized in that** the coronavirus infection disease comprises coronaviral inflammation.

10. The cyclic dinucleotide metal compound for use according to any one of claims 4-9, **characterized in that** a dosage form of the cyclic dinucleotide metal compound is tablet, capsule, enteric-coated particle, granule, suspension, emulsion, solution, syrup or inj ection.
